# EUROPEAN PATENT APPLICATION

(11) **EP 0 881 493 A1**
(43) Date of publication of application: **02.12.1998**
(21) Application number: 97941250.9
(22) Date of filing: 26.09.1997
(51) Int. Cl.: G01N 33/552

(54) **CARRIER FOR IMMUNOASSAY AND METHOD OF IMMUNOASSAY THEREWITH**

(30) Priority: 27.09.1996 JP 277154/96
(71) Applicant: SRL, INC., Tachikawa-shi, Tokyo 190 (JP)
(72) Inventor: KUMAZAWA, Toshiaki, 51, Komiya-cho Hachioji-shi Tokyo 192 (JP); TAGAMI, Hiroaki, 51, Komiya-cho Hachioji-shi Tokyo 192 (JP); KIYA, Yoshiyasu, 51, Komiya-cho Hachioji-shi Tokyo 192 (JP)
(74) Representative: Armitage, Ian Michael
(86) International application number: JP9703428
(87) International publication number: WO9813691

(57) **Abstract**

A carrier for immunoassays with which immunoassays can be carried out in a shorter time using smaller amounts of washing solution and sample than before is disclosed. The carrier for immunoassays according to the present invention consists essentially of glass fibers having an SiO₂ content of 40-95% by weight and an Na₂O content of 5-60% by weight.

## Description

### TECHNICAL FIELD

The present invention relates to a carrier for immunoassays and a method for immunoassay using the same.

### BACKGROUND ART

Conventionally, antigens and antibodies are widely measured by immunoassays, and methods employing solid carriers are widely employed among the immunoassays. Solid carriers used in immunoassays include polystyrene beads and wells of microplates.

One of the major methods employing a solid carrier is the enzyme immunoassay by sandwich method (sandwich ELISA). In cases where anti-Hepatitis B virus antigen, for example, is measured by this method, the Hepatitis B surface antigen (HBs antigen) is immobilized on a carrier and a sample serum dissolved in a reaction medium is reacted with the immobilized antigen. The components which did not react with the antigen and the antibody bound to the carrier by antigen-antibody reaction are then separated (B-F separation). Thereafter, anti-human IgG antibody labeled with an enzyme such as peroxidase (POD) is added to sandwich the bound antibody between the antigen immobilized on the carrier and the labeled anti-IgG antibody, thereby specifically detecting the bound antibody. The labeled antibody which did not participate in the reaction is removed by B-F separation. By adding a substrate of POD, the reaction mixture is colored depending on the POD activity, and the amount of the antibody is determined based on a calibration curve.

In the conventional immunoassays, the time required for the reaction between the immobilized antigen and the antibody in the serum is about 30 to 180 minutes and the time required for the reaction between the bound antibody and the labeled antibody is also about 30 to 180 minutes, so that the reactions are considerably time-consuming. Further, a large amount of washing solution as large as 1000 to 10,000 µl is required for the B-F separation.

### DISCLOSURE OF THE INVENTION

An object of the present invention is to provide means by which immunoassays may be carried out in a shorter time using a smaller amount of washing solutions and samples than the conventional immunoassays.

The present inventors intensively studied to discover that by using glass fibers having a specific composition as a carrier, the time for immune reactions may be largely shortened and the amounts of the washing solutions and samples may be largely reduced when compared with the conventional immunoassays, thereby completing the present invention.

That is, the present invention provides a carrier for immunoassays consisting essentially of glass fibers having an SiO₂ content of 40-95% by weight and an Na₂O content of 5-60% by weight. The present invention also provides use of glass fibers having an SiO₂ content of 40-95% by weight and an Na₂O content of 5-60% by weight as a carrier for immunoassays. Further, the present invention provides a method for immunoassay comprising using an immunologically active substance immobilized on said carrier for immunoassays according to the present invention.

By the present invention, a carrier for immunoassays, with which immunoassays can be carried out in a shorter time and the amounts of the washing solutions and samples are smaller than the conventional immunoassays, as well as a method for immunoassay using the carrier was provided. As will be described in the Examples, by the sandwich ELISA using the carrier for immunoassays according to the present invention, the immunoassays were able to be carried out in about 5 minutes. Further, the amounts of the used samples and washing solutions were smaller than the conventional methods. In spite of the fact that the reaction time is shorter and the amounts of the samples and the washing solutions were smaller, the sensitivities of the immunoassays were higher than the conventional ELISA. Thus, by the present invention, immunoassays can be carried out more quickly and more sensitively. Further, in cases where a microplate is used, a plate washer and a plate reader are necessary. However, with the method of the present invention, no special devices are necessary, so that the immunoassay may be carried in emergency or in developing areas where electricity is not available.

### BEST MODE FOR CARRYING OUT THE INVENTION

As mentioned above, the carrier for immunoassays according to the present invention consists essentially of glass fibers. The SiO₂ content of the glass fibers is 40-95% by weight, preferably 55-70% by weight, and the Na₂O content thereof is 5-60% by weight, preferably 10-20% by weight. If the SiO₂ and Na₂O contents are outside the above-described ranges, the effects of the present invention cannot be obtained.

The glass fibers used in the present invention may consist of only SiO₂ and Na₂O, and may contain components other than these components. Examples of the other components include P₂O₃, Al₂O₃, K₂O, CaO, MgO and the like. It should be noted, however, the glass fibers may contain one or more components other than the components mentioned above as long as the effects of the present invention are not lost. The contents of the third components such as P₂O₃, Al₂O₃, K₂O, CaO and MgO may preferably be 0-10% by weight, respectively. Especially preferred glass fibers include those containing 55-70% by weight of SiO₂, 12-18% by weight of Na₂O, 5-7% by weight of P₂O₃, 4-6% by weight of Al₂O₃, 0-2% by weight of K₂O, 5-7% by weight of CaO and 2-4% by weight of MgO.

Although the diameters of the glass fibers are not restricted, the diameters may preferably be 0.5-2.0 µm. The glass fibers may be in any form and those in the form of membrane are easy to use and preferred. In this case, although the basis weight of the membrane is not restricted, the basis weight may preferably be 90-130 g/m². The thickness of the membrane is also not restricted, and may preferably be 0.3-0.6 mm. Some glass fibers are commercially available, and among the commercially available glass fibers, those satisfying the requirements of the present invention may be used as the carrier for immunoassays according to the present invention.

The carrier for immunoassays according to the present invention may be employed for any immunoassays using a solid carrier. The method for immunoassay *per se* using the carrier of the present invention is basically the same as the conventional immunoassays. That is, on the carrier for immunoassays according to the present invention, an immunologically active substance is immobilized. The term "immunologically active substance" herein means any substance which may undergo antigen-antibody reaction, and includes not only antigens and antibodies, but also fragments thereof, for example, haptens, Fab fragments and F(ab')₂ fragments of antibodies. The carrier for immunoassays according to the present invention on which such an immunologically active substance is immobilized may be used for, for example, sandwich method in the same manner as the conventional carrier.
In cases where the carrier is used for sandwich method, the carrier according to the present invention on which an immunologically active substance is immobilized is blocked with a protein such as casein or bovine serum albumin (BSA) and a sample is reacted with the resultant. After washing the carrier, a second antibody is reacted with the resultant. After washing the resultant, the second antibody remained on the solid is measured. The measurement of the second antibody may be accomplished by preliminarily labeling the second antibody with an enzyme, fluorescent pigment, biotin, radioactive substance or the like and detecting the label.

Surprisingly, by using the carrier for immunoassays according to the present invention, the antigen-antibody reactions in immunoassays, that is, in case of the sandwich method, the reaction between the immunologically active substance immobilized on the carrier and the sample, and the reaction between the substance to be measured in the sample and the second antibody, may be carried out in a time much shorter than in the conventional methods. For example, in the Examples described below, the reaction time of antigen-antibody reactions was only 2 minutes, which is only one-several tensth of the reaction time in the conventional methods. Further, the amounts of the washing solution and the sample may be about half to one-fifth of those in the conventional methods. Although the reason why the antigen-antibody reaction proceeds much more quickly than in the conventional methods is not clearly understood, it is presumed that one of the reasons is that a large amount of the immunologically active substance is immobilized on the carrier. It is presumed that because of this, non-specific binding of the sample and the second antibody to the carrier is reduced, and so the required amount of the washing solution is small, and that the measuring sensitivity is increased and so the amount of the sample can be small.

The present invention will now be described more concretely by way of examples. It should be noted, however, the present invention is not restricted to the examples below.

### Example 1 Measurement of Anti-HCV NS5 Antibody by Sandwich ELISA

As a carrier for immunoassay, a glass fiber membrane, GA100 commercially available from ADVANTEC, which was sized 5 mm x 5 mm was used. The composition of the glass fibers constituting GA100 is shown in Table 1 below. The diameters of the glass fibers constituting GA100 were 0.8-1.5 µm. The basis weight of GA100 was 110 g/m², and the thickness of GA100 was 0.45 mm.

**Table 1**

| Component | Content (wt%) |
|---|---|
| SiO₂ | 63 |
| Na₂O | 15 |
| P₂O₃ | 6 |
| Al₂O₃ | 5 |
| K₂O | 1 |
| CaO | 6 |
| MgO | 3 |

HCV peptide antigen NS5b was dissolved in 0.01 M PBS (pH7) to a concentration of 10 µg/ml and the solution was incubated with the carrier at 4°C for 2 hours, thereby immobilizing the antigen on the carrier. Thereafter, 300 µl of 1% casein in 0.01 M PBS (pH7) was dropped on the carrier. The resulting membrane on which the antigen was immobilized and which was blocked was placed on a Kim Towel commercially available from JUJO KIMBERLY and the following operations were carried out.

As the test sera, sera from thirty HCV RNA-positive patients were used. Each of the test sera was 100-fold diluted with 0.01 M phosphate buffer containing 0.5 M NaCl and 1% BSA and 30 µl aliquot of the resultant was dropped on the carrier. After allowing to react at room temperature for 2 minutes, 300 µl of 0.01 M PBS (pH7) containing 0.05% Tween 20 (trademark) was dropped to carry out B-F separation. A solution containing 1 µg/ml of POD-labeled anti-human IgG antibody commercially available from CAPEL, 0.5 M NaCl and 1% BSA in 0.01 M PBS (pH7) was prepared, and 30 µl aliquot thereof was dropped. After allowing to react at room temperature for 2 minutes, 300 µl per time of 0.01 M PBS (pH7) containing 0.05% Tween 20 (trademark) was dropped twice, thereby carrying out B-F separation. Then 100 µl of 0.1 M citrate buffer containing 0.033% hydrogen peroxide and 1 mg/ml o-phenylenediamine was added and judgment was carried out one minute later. The judgment was carried out by comparing the color with those of the positive and negative controls. The positive samples were defined as those which exhibited color more strongly than the negative control and the negative samples were defined as those which exhibited color about equal to or weaker than the negative control. Among the 30 samples, 22 samples were positive.

### Comparative Example 1 Measurement of Anti-HCV NS5 Antibody by Sandwich ELISA Using Wells of Microplate as Carrier

The same antigen as used in Example 1 was dissolved in 0.01 M PBS (pH7) and the obtained solution was added to wells of a microplate 473709 commercially available from NUNC in an amount of 0.1 µg antigen/well, followed by incubation at room temperature for 1 hour to immobilize the antigen. Blocking was then carried out with 0.01 M PBS (pH7) containing 0.05% gelatin.

As the test sera, the same 30 sera as used in Example 1 were used. To each well of the microplate, a test serum which was 100-fold diluted with 0.01 M PBS containing 0.5 M NaCl and 1% BSA was added and the resultant was allowed to react at room temperature for 30 minutes. Washing for B-F separation was then performed 5 times using 300 µl each of Microplate Washer Cellawasher Auto II commercially available from SANKO JUNYAKU. To each well, 100 µl of 0.01 M PBS (pH7) containing 1 µg/ml of POD-labeled anti-human IgG antibody commercially available from CAPEL, 0.5 M NaCl and 1% BSA was added and the resultant was allowed to react at room temperature for 30 minutes. Each well was then washed 5 times with 300 µl per time of washing solution, and 100 µl of 0.1 M citrate buffer containing 0.033% hydrogen peroxide and 1 mg/ml o-phenylenediamine was then added to each well. Fifteen minutes later, 100 µl of 2N sulfuric acid was added to stop the reaction and absorbance at 420 nm was measured. The samples which exhibited an absorbance of more than 0.400 were judged as positive. Among the 30 samples, 18 samples were positive.

Example 1 and Comparative Example 1 will now be compared. In Comparative Example 1, the reaction times of the antigen-antibody reaction between the immobilized antigen and the antibody in the test serum, and of the antigen-antibody reaction between the antibody in the serum bound to the carrier and the POD-labeled anti-human IgG antibody were both 30 minutes. In contrast, in Example 1, these reaction times were both only 2 minutes. Therefore, the times required for the antigen-antibody reactions in Example 1 were as short as only one fifteenth of those required in Comparative Example 1. Further, the amount of the washing solution used in Comparative Example 1 was 1500 µl (300 µl x 5 times) for both reactions, while in Example 1, it was 300 µl (300 µl x once) or 600 µl (300 µl x twice). Thus, the number of times of washing operations is also much smaller in Example 1 than in Comparative Example 1. Further, the amount of the diluted test serum was 100 µl in Comparative Example 1, and was 30 µl in Example 1. Thus, in Example 1, the reaction times of the antigen-antibody reactions were as short as one fifteenth of those required in Comparative Example 1, and the amounts of the washing solutions, the number of washing operations and the amount of the test serum were smaller in Example 1 than in Comparative Example 1. In spite of this, the number of positive sera was 22 in Example 1 while it was 18 in Comparative Example 1, so that the measuring sensitivity is higher in Example 1 than in Comparative Example 1.

### Example 2 Measurement of Anti-HCV NS4 Antibody by Sandwich ELISA

On the same carrier for immunoassays as used in Example 1, HCV peptide antigen NS4 was immobilized, and anti-HCV NS4 antibody was measured in the same manner as in Example 1. As the test sera, HCV RNA-positive sera from 5 patients were used. For immobilizing the antigen on the carrier, a solution containing 10 µg/ml antigen (Method A) or a solution containing 1 µg/ml antigen (Method B) in 0.01 M PBS (pH7) was used. Except this, all other procedures were the same as in Example 1. As a result, 5 samples among the 5 samples were positive by Method A, and 3 samples among the 5 samples were positive by Method B.

### Comparative Example 2 Measurement of Anti-HCV NS4 Antibody by Sandwich ELISA Using Glass Fibers Outside Scope of the Invention as Carrier

The same procedure as in Example 2 was repeated except that glass fiber membrane GF/D commercially available from Whatman was used as the carrier. The chemical composition of GF/D was 99% SiO₂. The basis weight was 120 g/m² and the thickness was 0.68 mm. As a result, all of the samples were positive (i.e., 5 samples among the 5 positive samples were positive, and 5 samples among the 5 negative samples were positive), so that measurement cannot be attained by any of Method A and Method B.

### Example 3 Measurement of Anti-Treponema pallidum Antibody by Sandwich ELISA

The same carrier for immunoassays as in Example 1 was used. Native *Treponema pallidum* antigen (TP antigen) was dissolved in 0.01 M PBS (pH7) to a concentration of 10 µg/ml, and 30 µl aliquot of the obtained solution was added to the carrier, followed by incubation at room temperature for 15 minutes. Then 100 µl of 10% goat serum in 0.01 M PBS (pH7) was added to carry out blocking. The resulting membrane on which the antigen was immobilized and which was blocked was placed on a Kim Towel commercially available from JUJO KIMBERLY and the following operations were carried out.

As the test sera, 15 positive sera and 5 negative sera, judged by TPHA method were used. Each of the test sera was 200-fold diluted with 0.01 M phosphate buffer containing 0.5 M NaCl and 1% BSA and 30 µl aliquot of the resultant was dropped on the carrier. After allowing to react at room temperature for 90 seconds, 50 µl of 0.01 M PBS (pH7) containing 0.05% Tween 20 (trademark) was dropped to carry out B-F separation. A solution containing 1 µg/ml of POD-labeled anti-human IgG antibody commercially available from CAPEL, 0.5 M NaCl and 1% BSA in 0.01 M PBS (pH7) was prepared, and 30 µl aliquot thereof was dropped. After allowing to react at room temperature for 90 seconds, 100 µl x twice of 0.01 M PBS (pH7) containing 0.05% Tween 20 (trademark) was dropped, thereby carrying out B-F separation. Then 100 µl of 0.1 M citrate buffer containing 0.033% hydrogen peroxide and 1 mg/ml o-phenylenediamine was added and judgment was carried out 30 seconds later. The judgment was carried out by comparing the color with those of the positive and negative controls. The positive samples were defined as those which exhibited color more strongly than the negative control and the negative samples were defined as those which exhibited color about equal to or weaker than the negative control. As a result, among the 15 positive samples, 15 samples were positive, and among the 5 negative samples, 5 samples were negative. In spite of the fact that the measurement was carried out in a much shorter time than the TPHA method (the time required for TPHA method is 2 hours), the measuring sensitivity was the same as the TPHA method, so that the measurement was able to be carried out quickly.

### Comparative Example 3 Measurement of Anti-Treponema pallidum Antibody by Sandwich ELISA Using Glass Fibers Outside Scope of the Invention as Carrier

The same procedure as in Example 3 was repeated except that the carrier for immunoassays used in Comparative Example 2 was used. As a result, among the 15 positive samples, 15 samples were positive, and among the 5 negative samples, 5 samples were positive. Therefore, by this method, correct judgement could not be attained.

### Example 4 Measurement of Human IgE by Sandwich ELISA

The same carrier for immunoassays as used in Example 1 was used. Anti-human IgE antibody was dissolved in 0.01 M PBS (pH7) to a concentration of 10 µg/ml, and 30 µl aliquot of the obtained solution was added to the carrier, followed by incubation at room temperature for 120 minutes. Then 100 µl of 1% casein in 0.01 M PBS (pH7) was added to carry out blocking. The resulting membrane on which the antigen was immobilized and which was blocked was placed on a Kim Towel commercially available from JUJO KIMBERLY and the following operations were carried out.

As the test sera, 15 positive sera and 5 negative sera, judged by RAST method were used. Each of the test sera was 10-fold diluted with 0.01 M phosphate buffer containing 0.5 M NaCl and 1% BSA and 30 µl aliquot of the resultant was dropped on the carrier. After allowing to react at room temperature for 10 minutes, 50 µl of 0.01 M PBS (pH7) containing 0.05% Tween 20 (trademark) was dropped to carry out B-F separation. A solution containing 1 µg/ml of POD-labeled anti-human IgE antibody commercially available from DAGO, 0.5 M NaCl and 1% BSA in 0.01 M PBS (pH7) was prepared, and 30 µl aliquot thereof was dropped. After allowing to react at room temperature for 10 minutes, 100 µl x twice of 0.01 M PBS (pH7) containing 0.05% Tween 20 (trademark) was dropped, thereby carrying out B-F separation. Then 100 µl of 0.1 M citrate buffer containing 0.033% hydrogen peroxide and 1 mg/ml o-phenylenediamine was added and judgment was carried out 60 seconds later. The judgment was carried out by comparing the color with those of the positive and negative controls. The positive samples were defined as those which exhibited color more strongly than the negative control and the negative samples were defined as those which exhibited color about equal to or weaker than the negative control. As a result, among the 15 positive samples, 15 samples were positive, and among the 5 negative samples, 5 samples were negative. In spite of the fact that the measurement was carried out in a much shorter time than the RAST method (the time required for RAST method is 4 hours), the measuring sensitivity was the same as the RAST method, so that the measurement was able to be carried out quickly.

### Comparative Example 4 Measurement of Human IgE Antibody by Sandwich ELISA Using Glass Fibers Outside Scope of the Invention as Carrier

The same procedure as in Example 4 was repeated except that the carrier for immunoassays used in Comparative Example 2 was used. As a result, among the 15 positive samples, 15 samples were positive, and among the 5 negative samples, 5 samples were positive. Therefore, by this method, correct judgement could not be attained.

## Claims

1. A carrier for immunoassays consisting essentially of glass fibers having an SiO₂ content of 40-95% by weight and an Na₂O content of 5-60% by weight.

2. The carrier for immunoassays according to claim 1, wherein said SiO₂ content is 55-70% by weight and said Na₂O content is 10-20% by weight.

3. The carrier for immunoassays according to claim 1 or 2, wherein said glass fibers contain 0-10% by weight of P₂O₃, 0-10% by weight of Al₂O₃, 0-10% by weight of K₂O, 0-10% by weight of CaO and 0-10% by weight of MgO.

4. The carrier for immunoassays according to claim 3, wherein said glass fibers contain 55-70% by weight of SiO₂, 12-18% by weight of Na₂O, 5-7% by weight of P₂O₃, 4-6% by weight of Al₂O₃, 0-2% by weight of K₂O, 5-7% by weight of CaO and 2-4% by weight of MgO.

5. The carrier for immunoassays according to any one of claims 1-4, wherein said glass fibers have diameters of 0.5-2.0 µm.

6. The carrier for immunoassays according to any one of claims 1-5, which is in the form of a membrane consisting essentially of said glass fibers.

7. The carrier for immunoassays according to claim 6, wherein said membrane has a basis weight of 90-130 g/m².

8. The carrier for immunoassays according to claim 6 or 7, wherein said membrane has a thickness of 0.3 mm to 0.6 mm.

9. The carrier for immunoassays according to any one of claims 1-8, which is a carrier for sandwich method.

10. A method for immunoassay comprising using an immunologically active substance immobilized on said carrier for immunoassays according to any one of claims 1-9.

11. The method according to claim 10, which is carried out by sandwich method.

12. The method according to claim 10, which is carried out by sandwich ELISA.

13. The method according to any one of claims 10-12, which is for measuring anti-*Treponema pallidum* antibody.

14. The method according to any one of claims 10-12, which is for measuring IgE.

15. The method according to any one of claims 10-12, which is for measuring anti-HCV antibody.

16. The method according to claim 15, which is for measuring anti-HCV NS5 antibody.

17. The method according to claim 15, which is for measuring anti-HCV NS4 antibody.
